# NEUE EUROPÄISCHE PATENTSCHRIFT

(11) **EP 1 487 502 B2**
(45) Veröffentlichungstag und Bekanntmachung des Hinweises auf die Entscheidung über den Einspruch: **13.08.2008**
(45) Hinweis auf die Patenterteilung: 11.01.2006
(21) Anmeldenummer: 02792671.6
(22) Anmeldetag: 18.12.2002
(51) Int. Cl.: A61L 2/14, A61L 2/26, A61J 9/08, B65B 55/10

(54) **VERFAHREN UND VORRICHTUNG ZUMINDEST ZUR STERILISATION VON VERSCHLIESSELEMENTEN FÜR BEHAELTNISSE**
METHOD AND DEVICE AT LEAST FOR THE STERILISATION OF CLOSING ELEMENTS OF CONTAINERS
PROCEDE ET DISPOSITIF PERMETTANT AU MOINS LA STERILISATION D'ELEMENTS DE FERMETURE DE RECIPIENTS

(30) Priorität: 19.03.2002 DE 10211976
(43) Veröffentlichungstag der Anmeldung: 22.12.2004
(73) Patentinhaber: ROBERT BOSCH GMBH, 70442 Stuttgart (DE)
(72) Erfinder: GOETZELMANN, Bernd, 74585 Rot Am See (DE); RAUSCHNABEL, Johannes, 70197 Stuttgart (DE); HAEGELE, Hartmut, 71701 Schwieberdingen (DE); WILKE, Bernd, 71397 Leutenbach (DE); BURGER, Kurt, 71292 Friolzheim (DE)
(86) Internationale Anmeldenummer: PCT/DE2002/004628
(87) Internationale Veröffentlichungsnummer: WO 2003/077959

(56) Entgegenhaltungen:
- EP-A- 0 377 799
- WO-A-96/13337
- WO-A-03/016143
- DE-A- 19 937 008
- US-A- 2 095 502
- US-A- 3 737 608
- US-B1- 6 230 472

## Beschreibung

### Stand der Technik

Die Erfindung betrifft ein Verfahren zumindest zur Sterilisation von Behältnissen und/oder deren Verschließelementen und eine Vorrichtungen zur Durchführung des Verfahrens mit einer Plasmabehandlung, ausgehend von den gattungsgemäßen Merkmalen der Haupt- und Nebenansprüche.

Es ist allgemein bekannt, zur Beseitigung von schädlichen Mikroorganismen oder Keimen in Behältnissen in der Medizin oder der Lebensmitteltechnologie, z.B. bei Ampullen, Schnappdeckelgläser, Septengläser oder sog. Vials bzw. sonstigen sog. Parenteralia-Verpackungen physikalische oder chemische Verfahren einzusetzen.

Im Bereich der Medizintechnik ist ein Verfahren beispielsweise aus der US-PS 5,961,921 bekannt, bei dem medizinische Instrumente mittels H₂O₂-Dampf im Vakuum sterilisiert werden und mit einem Hochfrequenzplasma anschließend die Peroxidreste entfernt werden. Ein ähnliches Verfahren ist im Bereich der Getränkeabfüllung bekannt, bei dem sog. PET-Flaschen vor der Befüllung im Vakuum mit H₂O₂ sterilisiert werden und die Entfernung der Peroxidreste hierbei durch eine Absenkung des Vakuums erreicht wird.

Weiterhin ist aus der US-PS 6,230,472 auch die Plasmasterilisation von Stopfen als Verschließelemente für einen Behälter bekannt, und zwar im Rahmen eines Prozesses, bei dem der Behälter, die Füllnadel, der Verschluss und eine Vakuumkammer gleichzeitig mittels Niederdruck- und Niedertemperaturplasma sterilisiert werden.

Darüber hinaus ist noch aus der WO 96/13337 eine Vorrichtung zur Plasmabeschichtung von Verschließelementen für Vials bekannt, die im Niederdruck innerhalb einer Trommel erfolgt. Dieses Verfahren ist wegen der Trommelgeometrie, wegen des Schüttgut-Prinzips und dem diskontinuierlichen sog. Batch-Verfahren insbesondere für eine Abwandlung in Richtung einer oben erwähnten Sterilisation und einer häufig notwendigen Entpyrogenisierung, ungeeignet und beschränkt sich außerdem lediglich auf eine Beschichtung der Verschlussstopfen.

Nachteilig ist bei diesen bekannten Verfahren insbesondere die Komplexität des Sterilisationsprozesses, besonders hinsichtlich der Geometrie der zu sterilisierenden Elemente, die Hohlformen, Hinterschneidungen, Halterungen etc. aufweisen. Ein weiterer Nachteil ist bei den hierzu bekannten Vorrichtungen auch die sequentielle Vorgehensweise zum Sterilisieren, Befüllen und Verschließen der Behältnisse, die hier zwar mit allen Arbeitsgängen in derselben Kammer durchgeführt werden müssen, jedoch wegen des Zusammenspiels von Druck, Plasmaanregung und Befüllung durch verfahrbare Füllnadel hintereinander ablaufen müssen.

Um einen wirtschaftlichen Sterilisations- und darüber hinausgehenden Pyrogenabreicherungsprozess bei verschließbaren Behältnissen zu gewährleisten, sollten kurze Prozesszeiten mit kostengünstigen Vorrichtungen erzielt werden. Darüber hinaus müssen zumindest im pharmazeutischen Bereich diese Prozesse nach einer vorgegebenen Norm vollständig validierbar sein und möglichst über eine sog. in-situ Prozesskontrolle verfügen.

Zusammenfassend betrachtet sieht die bisherige Praxis z.B. bei Vials und Injektionsflaschen in der Medizin vor allem eine separate Sterilisation und Vergütung von Behälter und Verschließelement vor. Die Behälter werden z.B. mittels Hitze sterilisiert und entpyrogenisiert, während die Verschlusselemente, z.B. Stopfen oder Septen, in der Regel zur Sterilisation in einem Autoklaven mit Sattdampf bei ca. 121°C beaufschlagt werden. Eine signifikante Pyrogenabreicherung kann im Autoklaven nicht erfolgen. Auch vorhergehende oder nachfolgende wässrige Reinigungsschritte erzielen keine ausreichende Abreicherung. Chemische Entpyrogenisierung, z.B. mit starken Säuren oder Alkalien verändern die Elastomere der Verschließelemente in ungewünschter Weise und kommen daher kaum zur Anwendung.

Die zuvor erwähnte Autoklavierung der Elastomer-Verschließelemente im Schüttgut außerhalb des Isolators ist auch deswegen nachteilig, da in der Regel die dann erforderliche Zuführung zur Verschließmaschine notwendigerweise eine Vereinzelungsstation beinhaltet, bei der eine schädliche Partikelgeneration nicht ganz auszuschließen ist.

Ein Verfahren zur Sterilisation von Behältnissen der zuvor genannten Art und eine Vorrichtungen zur Durchführung des Verfahrens mit einer Plasmabehandlung der Behältnisse in einer Vakuumkammer ist außerdem in der nicht vorveröffentlichten DE 101 38 938 beschrieben.

Aus der US-A-2095502 sind ein Verfahren und eine Vorrichtung zum Sterilisieren von Tellern und Besteck durch Bestrahlung mit Licht bekannt, wobei die zu sterilisierenden Teile auf einem Förderband liegen und während der Bestrahlung so gedreht werden, dass jede Seite der Teile dem Licht ausgesetzt wird. Aus der US-A-3737608 ist ein Verfahren zur Plasmasterilisation und anschließende Befüllung eines Behälters innerhalb der Sterilisationskammer bekannt.

### Vorteile der Erfindung

Mit der Erfindung wird ein Verfahren und einen Vorrichtung zur Sterilisation und/oder Entpyrogenisierung von Verschließelementen für Behältnisse der eingangs angegebenen Art und insbesondere auch im Zusammenhang mit der Sterilisation und/oder Entpyrogenisierung der Behältnissen selbst in vorteilhafter Weise mit den Merkmalen des unabhängigen Anspruchs 1 fortgebildet. Dies geschieht unter Ausnutzung eines oder mehrerer Plasmen, wobei insbesondere Niederdruckplasmen, atmosphärendrucknahe oder atmosphärische Plasmen hier vorteilhaft anwendbar sind.

Es ist somit bei Benutzung des erfindungemäßen Verfahrens möglich, eine Vorrichtung zu gestalten, die parallel zum Sterilisations-, Pyrogenabreicherungs- und ggf. Vergütungsprozess für die Behältnisse einen solchen Prozess auch für die Verschließelemente, insbesondere für solche aus Elastomermaterialien, enthält und damit insgesamt vollständig validierbare Prozesse ermöglicht. Bei einer Anwendung im Medizin- oder Lebensmittelbereich sollte aus Gründen der hier notwendigen Zulassungsverfahren, insbesondere auch wegen der Flexibilität in der Zusammenstellung einer Abfülllinie für spezielle Kundenwünsche, daher ein modulares Konzept der Sterilisation von Behälter und Verschließelement zum Befüllen und Verschließen angestrebt werden.

Insbesondere muss für die eingangs angegebenen Verfahren sichergestellt.werden, dass alle Bereiche und Flächen der Verschließelemente steril sind, wenn Sie in die Abfüllmaschine überführt werden. Es darf keine Kreuzkontamination von Behältern, Verschließ- oder Handlingselementen erfolgen können. Jede Partikelgeneration, z.B. durch Stoß oder Abrieb, muss möglichst vermieden werden, wobei gemäß der Erfindung eine klare Sterilgrenze in einer Verpackungslinie für die erwähnten Behältnisse realisiert ist, hinter der alles steril ist, bzw. steril gehalten ist und eine Rekontamination weitgehend ausgeschlossen werden kann.

In den Ansprüchen 1 bis 4 sind daher Verfahrensmerkmale angegeben, mit denen in besonders vorteilhafter Weise eine zur Behältersterilisation und Pyrogenabreicherung parallele Behandlung der Verschließelemente, insbesondere von Elastomerstopfen, mittels Anregung eines Plasmas in einer Vakuumkammer unter Einhaltung der zuvor erwähnten Randbedingungen möglich ist.

Dabei werden die Verschließelemente einzeln geführt und auf ihrem Weg durch die Sterilisationskammer auf allen Seiten sterilisiert und es werden auf dem Verschließelement haftende Pyrogene um Dekaden abgereichert, was mit den üblichen bekannten Verfahren nicht möglich ist. Durch diese weitgehend vereinzelte Handhabung wird eine Partikelgenerierung wenn nicht ausgeschlossen, so doch zumindest minimiert. Im Falle der Sterilisation in einer Vakuumkammer bei Drücken unter 1 mbar kann eine Partikelverschleppung in die Füllmaschine fast ausgeschlossen werden. Die vereinzelte Führung der Verschließelemente erlaubt auch auf einfache Weise Element für Element eine Prozesskontrolle und -dokumentation.

Eine für viel Anwendungsfälle auch vorteilhafte parallele Anordnung der Verschließelement- zur Behältersterilisation in einer oder in benachbarten besonderen Vakuumkammern ermöglicht eine kleinere Baugröße der Gesamtanlage und eine kostengünstigere Vorrichtung als bei Konzepten, in denen sequentiell die Stationen Plasmasterilisation, Befüllung und Verschließen in ein und derselben Kammer durchgeführt wird.

Nach der Einschleusung der Verschließelemente ins Vakuum der Kammer müssen die Elemente allseitig sterilisiert und entpyrogenisiert, ggf. auch vergütet oder beschichtet werden. Dadurch, dass in der erfindungsgemäßen Vorrichtung nicht nur die Verschließelemente, sondern auch die Halterungs- und Transporteinrichtungen ständig einem Plasma ausgesetzt sind, wird auch deren Sterilität sichergestellt und eine Kreuzkontamination vor der Ausschleusung vermieden.

Gemäß der mit den Ansprüchen 5 bis 9 vorgeschlagenen Ausführungsformen werden als Transporteinrichtungen Halterungen mit wechselndem Kontaktbereich zur Verschließelementeoberfläche über Stempel oder über Nadeln vorgesehen. Es ist jedoch auch eine Halterung anwendbar, die sich auch im Kontaktbereich selbst und auch die Berührungsfläche des Verschließelements ständig steril hält, z.B. durch eine hohe Temperatur.

Da hierbei die zu behandelnden Oberflächen der Stopfen für eine effiziente Sterilisation, Entpyrogenisierung und/oder Vergütung durch Vereinzelung und separate Behandlung auf einfache Weise in die optimale Position zur Plasmaquelle gebracht werden können, ist es möglich, Ausführungsformen für eine Halterung vorzusehen, bei denen nach einem ersten Schritt der Sterilisation eines Verschließelementebereichs dann in einem weiteren Schritt das Verschließelement an eben diesem nunmehr sterilen Bereich gefasst werden kann und zwar von einer sterilen Halterung, um dann die vorherige Halterung und den bis dahin unbehandelten Bereich zu sterilisieren. Dies bedingt aber, dass keine Rekontamination des bereits sterilen Stopfenbereichs durch die vorherige Halterung möglich ist.

Gemäß der Unteransprüche können jeweils vorteilhafte Anordnungen der Plasmaquelle im Inneren der Kammer angeordnet sein, wobei die Transportvorrichtung selbst mit einem elektrischen Anschluss, z.B. der Masse der Plasmaquelle verbunden sein kann.

Weiterhin ist es auch vorteilhaft möglich, dass die mindestens eine Plasmaquelle, z.B. ein sog. Gigatron als Koaxialantenne, im Inneren der Kammer entlang der Längsachse eines rotierenden und leicht geneigten Quarzrohres angebracht ist.

### Zeichnung

Ausführungsbeispiele von Vorrichtungen zur Durchführung des erfindungsgemäßen Verfahren insbesondere zur Sterilisation von Verschließelementen für Behältnisse werden anhand der Zeichnung erläutert. Es zeigen:
Figur 1 ein Blockschaltbild eines Verfahrensablauf zur Abfüllung von Behältnissen einschließlich der Sterilisation von Verschließelementen für die Behältnisse und der Behältnisse selbst,
Figur 2 ein erstes Ausführungsbeispiel einer Halterung mit einem vertikal verfahrbaren Stempel für ein einzelnes Verschließelement in der Vakuumkammer für eine Plasmabehandlung,
Figur 3 ein zweites Ausführungsbeispiel einer Halterung mit vertikal verfahrbaren Nadeln für ein einzelnes Verschließelement in der Vakuumkammer,
Figur 4 ein drittes Ausführungsbeispiel einer Halterung mit in Abänderung zur Figur 3 vertikal verfahrbaren Nadeln, die in Gruppen wechselseitig zueinander geneigt sind,

### Beschreibung der Ausführungsbeispiele

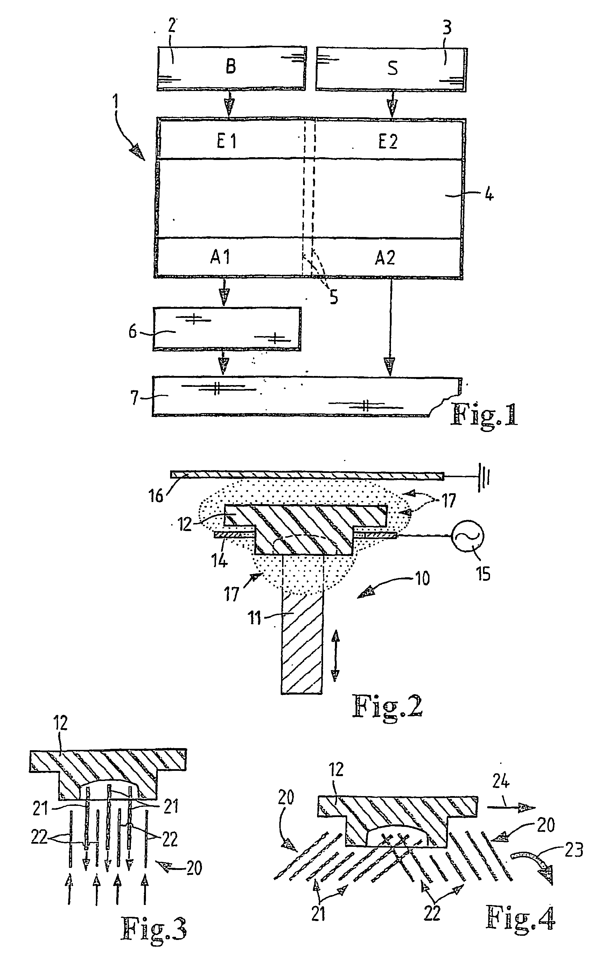

In Figur 1 ist ein Blockschaltbild 1 eines Verfahrens zur Sterilisation und/oder Entpyrogenisierung von hier nicht näher dargestellten Behältnissen in der Medizin oder der Lebensmitteltechnologie gezeigt, z.B. von Ampullen, Flaschen oder Gläsern und deren Verschließelementen, insbesondere Stopfen aus einem Elastomer.

Zunächst können hier gemäß Block 2 und 3 der Figur 1 die Behältnisse B und die Stopfen S separat vorgereinigt, z.B. gewaschen werden und dann jeweils in eine Niederdruck- oder Vakuumkammer E1 bzw. E2 eingeschleust werden, die hier als eine einzige Kammer dargestellt ist, wobei allerdings durch die gestrichelten Linen 5 angedeutet ist, dass die Vakuumkammer 4 auch für beide Verfahrensbereiche getrennt aufgebaut sein kann. Innerhalb der Kammer 4 wird dann eine Plasmabehandlung zur eigentlichen Sterilisation und/oder Entpyrogenisierung durchgeführt.

Eine solche Plasmabehandlung kann durch die Erzeugung einer elektromagnetischen Schwingung im Bereich von wenigen Hertz bis in den Mikrowellenbereich in der Niederdruck- oder Vakuumkammer 4 oder durch eine entsprechend geführte Welleneinkopplung in die Kammer 4 erfolgen. Für sich gesehen sind solche Plasmabehandlungsverfahren zur Sterilisation und/oder Vergütung aus dem in der Beschreibungseinleitung aufgeführten Stand der Technik bekannt.

Im Anschluss an die Plasmabehandlung kann nach dem Ausschleusen A1 bzw. A2 der Behältnisse und des Stopfens als Verschließelement aus der Kammer 4 ein Abfüllen des Behältnisses in einer Füllmaschine gemäß Block 6 erfolgen. Das gefüllte Behältnis und der Stopfen zum Verschließen des Behältnisses werden dann einer Verschließstation 7 zugeführt, in der das Behältnis dann endgültig verschlossen werden kann. Ziel des Verfahrens ist es, dass besonders während der Verfahrensschritte in der Kammer 4 nicht nur die Behältnisse und Verschließelemente, sondern auch die Halterungs- und Transporteinrichtungen ständig einem Plasma ausgesetzt sind, so wird auch deren Sterilität sichergestellt und eine Kreuzkontamination vor der Ausschleusung vermieden. Entsprechende Ausführungsbeispiele einer solchen Vorrichtung werden nachfolgend beschrieben.

Aus Figur 2 ist das erste Ausführungsbeispiel einer in einer Vakuumkammer angeordneten Transportvorrichtung zu entnehmen, die eine relativ feste Halterung 10 mit einem vertikal verfahrbaren Stempel 11 zur Aufnahme jeweils eines Elastomer-Stopfens 12 als Verschließelement für ein hier nicht dargestelltes Behältnis aufweist. Der Stopfen 12 wird mit seiner napfförmigen Innenseite so auf einem elektrisch leitenden Ring 14 platziert, dass die nach unten weisende Innenseite in den Ring.14 passt. Der Ring 14 wird mit einer Spannung eines Schwingungserzeugers 15 beaufschlagt, wobei als Gegenelektrode der Stempel 11 dient, der hier genauso auf Masse gelegt ist wie die über dem Ring platzierte Fläche 16.

Das Ausgangssignal des Schwingungserzeugers 15 wird zur Erzeugung eines Plasmas 17 dabei in an sich bekannter Weise im Vorzeichen und in der Amplitude moduliert, wobei die Modulation bei diesem Ausführungsbeispiel im Bereich von wenigen Hertz bis zu mehreren Hundert Megahertz reichen kann, bevorzugt aber zwischen 50 kHz und 27 MHz liegt. Speziell sind hierbei Schwingungen im Radiofrequenzbereich von 13,56 MHz und 27 MHz geeignet.

In einem ersten Schritt, nachdem der Stopfen 12 auf dem Ring 14 abgelegt worden ist, werden mit dem hier gepunktet angedeuteten Plasma 17 alle Seiten des Stopfens 12, bis auf die Kontaktfläche zwischen dem Ring 14 und dem Stopfen 12, mit diesem Plasma beaufschlagt. In einem zweiten Schritt wird der geerdete Stempel 11 unter dem Stopfen 12 vertikal nach oben bewegt, bis er den Stopfen 12 einige Millimeter aus dem Ring 14 gehoben hat. Dies ermöglicht dann die Sterilisation, die Entpyrogenisierung oder ev. auch eine Vergütung der bisher unbehandelten Kontaktflächen am Ring 14 und am Stopfen 12.

Dieser nun allseitig sterilisierte Stopfen 12 kann nun der Ausschleusung aus der Kammer zugeführt werden. Die Vorrichtung 10 nach der Figur 2 kann darüber hinaus noch dadurch verbessert werden, dass der Ring 14 mit einer dünnen, isolierenden Polymerschicht überzogen worden ist, da so eine reproduzierbar kapazitive Einkopplung der Energie sichergestellt werden kann und Lichtbögen, die an blanken Metallflächen unterschiedlicher Polung häufig auftreten, ausgeschlossen sind.

Bei einem Ausführungsbeispiel nach Figur 3 wird der Stopfen 12 in Abwandlung zur Halterung 10 nach der Figur 2 auf ein Nadelkissen 20 gebracht, bei dem eine Anzahl Nadeln 21 als Gruppe eine ausreichende Stabilität'der Halterung des Stopfens 12 zulassen und eine weitere Gruppe 22 anderer Nadeln dieselbe Aufgabe in einem anderen Verfahrensschritt übernehmen können.

Das Plasma wird hier in vergleichbarere Weise wie bei der Halterung 10 nach der Figur 2 erzeugt und beaufschlagt sowohl die Nadeln 21, bis auf deren Kontaktfläche mit dem Stopfen 12, als auch den Stopfen 12 selbst, wiederum bis auf dessen Kontaktflächen mit den Nadeln 21, mit dem Plasma. Um auch die zuvor genannten Kontaktflächen zu behandeln, werden die Nadeln 22 der weiteren Gruppe durch gegenseitiges vertikales Bewegen der Nadeln 21 und 22 in einem weiteren Schritt so unter den Stopfen 12 platziert, dass diese dann allein die Halterung des Stopfens 12 übernehmen. Die zuvor halternden Nadeln 21 werden dann in eine Position verfahren, in der sie mit Plasma beaufschlagt werden können, genauso wie die Bereiche des Stopfens 12, die mit Halterungsnadeln 21 Kontakt der ersten Gruppe gehabt haben und damit gegenüber dem Plasma abgeschirmt waren.

Die Bewegung der Nadeln 21 und 22 kann, wie erwähnt, entweder durch eine lineare Bewegung nach der Figur 3 und/oder durch eine Bewegung mit sich ändernder Neigung nach Figur 4 erfolgen. Bei dieser Art der Bewegung (Pfeil 23) der Gruppen 21 und 22 des Nadelkissens 20 kann eine gegen- und wechselseitige Neigung, ggf. zusammen mit einer linearen Bewegung, so erfolgen, dass der Stopfen 12 gleichzeitig eine Bewegung in horizontaler Richtung (Pfeil 24) vornimmt und damit die Vorrichtung als sog. Kammförderer fungiert.

Um eine möglichst allseitige Plasmabeaufschlagung des Stopfens 12 zu gewährleisten kann eine elektrische Kontaktierung der Nadeln nach den Figuren 3 und 4 mit dem Schwingungserzeuger 15 (vgl. Figur 2) vorgenommen werden. Um elektrische Kontakte zwischen mehreren Stopfen 12 in der Prozesslinie zu vermeiden, sollten diese einzeln oder mit einem vorgegebenen Abstand, also nicht im sog. Bulk, die Sterilisations-, Entpyrogenisierungs- und Vergütungsstrecke passieren.

Bei den zuvor beschriebenen Halterung nach den Figuren 2 bis 4 kann auch eine Parallelisierung in der Prozesslinie dadurch vorgenommen werden, dass anstelle' des Ringes 14 nach der Figur 2 ein Lochblech und ein Stempelkissen statt eines einzelnen Stempels 11 sowie eine Nadelkissen bei den Ausführungsbeispielen nach den Figuren 3 und 4 vorgesehen werden, um bei sicherer elektrischer Kontaktierung einen hohen Teiledurchsatz zu ermöglichen.

## Patentansprüche

1. Verfahren zumindest zur Sterilisation von Verschließelementen (12) für Behältnisse, bei dem
- in mindestens einem Verfahrensschritt in einer Niederdruck- oder Vakuumkammer (4) eine Plasmabehandlung durch Anregung einer elektromagnetischen Schwingung durchgeführt wird und bei dem
- das Plasma (17;47) in der Nähe des Verschließelements (12) oder einer Gruppe von Verschließelementen (12) und einer Transportvorrichtung (10;20;30;40,41;43) für die Verschließelemente (12) angeregt wird,
- wobei die zu sterilisierenden, zu entpyrogenisierenden und/oder zu vergütenden Bereiche des oder der Verschließelemente (12) zwischen dem Einschleusen und dem Ausschleusen in der Kammer (4) durch eine Bewegung im und durch das Plasma behandelt werden,
- wobei die Verschließelemente (12) einzelngeführt. und auf ihrem Weg durch die Kammer (4) auf allen Seiten sterilisiert werden.

2. Verfahren nach Anspruch 1, bei dem
- in mindestens einem Verfahrensschritt in einer Niederdruck- oder Vakuumkammer (4) eine Plasmabehandlung gemeinsam für die Behältnisse und die Verschließelemente (12) durch Anregung einer elektromagnetischen Schwingung gemeinsam durchgeführt wird und bei dem
- das Plasma (17;47) in der Nähe des Verschlie-ßelementes (12) oder einer Gruppe von Verschließelementen (12), einer Transportvorrichtung (10;20;30;40,41;43) und der Behältnisse angeregt wird, wobei
- die zu sterilisierenden, zu entpyrogenisierenden und/oder zu vergütenden Bereiche des oder der Verschließelemente (12) und der Behältnisse zwischen dem Einschleusen und dem Ausschleusen in der Kammer (4) durch eine Bewegung im und durch das Plasma behandelt werden.

3. Verfahren nach Anspruch 1, bei dem
- in mindestens einem Verfahrensschritt in jeweils einer Niederdruck- oder Vakuumkammer (4) eine Plasmabehandlung für die Behältnisse und in jeweils einer anderen Niederdruck- oder Vakuumkammer (4) die verschließelemente (12) durch Anregung einer elektromagnetischen Schwingung durchgeführt wird und bei dem
- das Plasma (17;47) jeweils in der Nähe jeweils eines oder einerGruppe von Verschließelementen (12) und einer Transportvorrichtung (10;20;30;40,41;43) und jeweils in der Nähe der Behältnisse und einer Transportvorrichtung angeregt wird, wobei
- die zu sterilisierenden und/oder zu entpyrogenisierenden Bereiche des oder der Verschließelemente (12) und der Behältnisse zwischen dem Einschleusen und dem Ausschleusen in den Kammern (4,5) durch eine Bewegung im und durch das Plasma behandelt werden.

4. Verfahren nach Anspruch 1, 2 oder 3, **dadurch gekennzeichnet, dass**
- vor dem Einschleusen der Behältnisse und der Verschließelemente (12) in die Kammern (4) eine Vorreinigung und/oder
- nach einer Ausschleusung der Behältnisse und der Verschließelemente (12) aus der oder den Kammern (4) ein Befüllen (6) der Behältnisse und jeweils ein Verschließen (7) der Behältnisse mit einem Verschließelement (12) erfolgt.

5. Vorrichtung zur Sterilisation von Verschließelementen (12) für Behältnisse mit einem Verfahren nach einem der vorhergehenden Ansprüche,
- mit einer Plasmaquelle (15) zur Erzeugung eines für eine Plasmabehandlung erforderlichen Plasmas,
- mit einer Niederdruck - oder Vakuumkammer (4), in der eine Plasmabehandlung durch Anregung einer elektromagnetischen Schwingung durchführbar ist,
wobei das Plasma (17; 47) in der Nähe des Verschließelements (12) oder einer Gruppe von Verschließelementen (12) und einer Transportvorrichtung (10) anregbar ist,
- wobei eine Einzelführung der Verschließelemente (12) vorgesehen ist, so dass die Verschließelemente (12) auf ihrem Weg durch die Kammer (4) auf allen Seiten sterilisiert werden, wobei
- in der Kammer die Transportvorrichtung (10) vorhanden ist, die eine Bewegung des oder der Verschließelemente (12) zum Zwecke des Transports von der Einschleusung (44) zur Ausschleusung (46) in der Kammer und dabei durch das Plasma hindurch bewirkt, wobei eine separate Vorreinigung (2) der Verschließelemente (12) vorgesehen ist.

6. Vorrichtung nach Anspruch 5, **dadurch gekennzeichnet, dass**
- die Transportvorrichtung (10) einen Stempel (11) aufweist, auf den jeweils ein Verschließelement (12) aufbringbar ist, wobei der Stempel (11) jeweils einen elektrischen Anschluss für die Plasmaquelle (15) darstellt und dass ein Ring (14), auf dem ein umlaufender Vorsprung des Verschließelements (12) aufliegt, den anderen elektrischen Anschluss für die Plasmaquelle (15) darstellt und dass
- der Stempel (11) vertikal so verfahrbar ist, dass jeweils abwechselnd das Verschließelement (12) entweder auf dem Stempel (11) oder auf dem Ring (14) aufliegt.

7. Vorrichtung nach Anspruch 5, **dadurch gekennzeichnet, dass**
- die Transportvorrichtung ein Nadellager oder
- kissen (20) für jeweils ein Verschließelement (12) aufweist und dass
- die Nadeln (21,22), auf denen des Verschließelement (12) aufliegt, im wesentlichen vertikal jeweils gruppenweise so verfahrbar sind, das jeweils abwechselnd das Verschließelement (12) entweder auf der einen Gruppe (21) oder auf der anderen Gruppe (22) der Nadeln aufliegt.

8. Vorrichtung nach Anspruch 7, **dadurch gekennzeichnet, dass**
- die Nadeln, auf denen des Verschließelement aufliegt, in den Gruppen (21,22) wechselweise gegenwinklig geneigt sind.und wechselweise linear und/oder hinsichtlich des Neigungswinkels jeweils teilweise so bewegbar sind, dass dabei auch eine Neigung und/odertranslatorische Bewegung des Verschließelements (12) durchführbar ist.

9. Vorrichtung nach einem der Ansprüche 6 bis 8, **dadurch gekennzeichnet, dass**
- zur Halterung mehrerer Verschließelemente (12) der Ring (14) Bestandteil eines Lochbleches, der Stempel (11) Bestandteil eines Stempelkissens oder die Nadeln (20,21,22) Bestandteil eines erweiterten Nadelkissen sind.

10. Vorrichtung nach einem der Ansprüche 7 bis 9, **dadurch gekennzeichnet, dass**
- die Nadeln (20,21,22) oder die Stempel (11) jeweils einen elektrischen Anschluss für die Plasmaquelle (15) darstellen.

11. Vorrichtung nach einem der Ansprüche 5 bis 10, **dadurch gekennzeichnet, dass**
- die mindestens eine Plasmaquelle im Inneren der Kammer angeordnet ist.

12. Vorrichtung nach einem der Ansprüche 5 bis 11, **dadurch gekennzeichnet, dass**
- die Transportvorrichtung mit einem elektrischen Anschluss der Plasmaquelle (15) oder einer Einrichtung zur Erzeugung eines elektrischen Vorspannung verbunden ist.

## Claims

1. Method at least for the sterilization of closing elements (12) for containers, in which,
- in at least one method step, plasma treatment is carried out in a low-pressure or vacuum chamber (4) by the excitation of an electromagnetic oscillation, and in which
- the plasma (17; 47) is excited in the vicinity of the closing element (12) or of a group of closing elements (12) and of a transport device (10; 20; 30; 40, 41; 43) for the closing elements (12),
- those regions of the closing element or closing elements (12) which are to be sterilized, to be depyrogenized and/or to be annealed being treated, between entry and exit, in the chamber (4) by means of a movement in and through the plasma,
- the closing elements (12) being guided individually and sterilized on all sides on their way through the chamber (4).

2. Method according to Claim 1, in which,
- in at least one method step, plasma treatment is carried out in a low-pressure or vacuum chamber (4) jointly for the containers and the closing elements (12) by the excitation of an electromagnetic oscillation, and in which
- the plasma (17; 47) is excited in the vicinity of the closing element (12) or of a group of closing elements (12), of a transport device (10; 20; 30; 40, 41; 43) and of the containers,
- those regions of the closing element or closing elements (12) and of the containers which are to be sterilized, to be depyrogenized and/or to be annealed being treated, between entry and exit, in the chamber (4) by means of a movement in and through the plasma.

3. Method according to Claim 1, in which,
- in at least one method step, plasma treatment for the containers is carried out in each case in one low-pressure or vacuum chamber (4) and plasma treatment for the closing elements (12) is carried out in each case in another low-pressure or vacuum chamber (4) by the excitation of an electromagnetic oscillation, and in which
- the plasma (17; 47) is excited in each case in the vicinity of in each case one or a group of closing elements (12) and a transport device (10; 20; 30; 40, 41; 43) and in each case in the vicinity of the containers and of a transport device,
- those regions of the closing element or closing elements (12) and of the containers which are to be sterilized and/or to be depyrogenized being treated, between entry and exit, in the chambers (4, 5) by means of a movement in and through the plasma.

4. Method according to Claim 1, 2 or 3, **characterized in that**
- before the entry of the containers and of the closing elements (12) into the chambers (4), a precleaning takes place and/or
- after an exit of the containers and of the closing elements (12) from the chamber or chambers (4), a filling (6) of the containers and in each case a closing (7) of the containers by means of a closing element (12) take place.

5. Device for the sterilization of closing elements (12) for containers by means of a method according to one of the preceding claims,
- with a plasma source (15) for generating a plasma required for plasma treatment,
- with a low-pressure or vacuum chamber (4) in which plasma treatment can be carried out by the excitation of an electromagnetic oscillation, the plasma (17; 47) being excitable in the vicinity of the closing element (12) or a group of closing elements (12) and a transport device (10),
- a guidance of the closing elements (12) being provided, so that the closing elements are sterilized on all sides on their way through the chamber (4).
- there being in the chamber the transport device which causes a movement of the closing element or closing elements (12) for the purpose of transport from the entry (44) to the exit (46) in the chamber and at the same time through the plasma,
- a separate precleaning (2) of the closing elements (12) being provided.

6. Device according to Claim 5, **characterized in that**
- the transport device (10) has a ram (11), onto which in each case a closing element (12) can be applied, the ram (11) constituting in each case one electrical connection for the plasma source (15), and **in that** a ring (14), on which a peripheral projection of the closing element (12) lies, constitutes the other electrical connection for the plasma source (15), and **in that**
- the ram (11) can be moved vertically in such a way that the closing element (12) in each case lies alternately either on the ram (11) or on the ring (14).

7. Device according to Claim 5, **characterized in that**
- the transport device has a needle bearing or needle cushion (20) in each case for a closing element (12), and **in that**
- the needles (21, 22) on which the closing element (12) lies can be moved essentially vertically in each case in groups in such a way that the closing element (12) lies in each case alternately either on one group (21) or on the other group (22) of needles.

8. Device according to Claim 7, **characterized in that**
- the needles on which the closing element lies are inclined in the groups (21, 22) alternately at opposite angles and can in each case be partially moved alternately linearly and/or with regard to the angle of inclination in such a way that in this case an inclination and/or a translational movement of the closing element (12) can also be carried out.

9. Device according to one of Claims 6 to 8,
**characterized in that**
- for holding a plurality of closing elements (12), the ring (14) is an integral part of a perforated plate, the ram (11) is an integral part of a ram cushion or the needles (20, 21, 22) are an integral part of a widened needle cushion.

10. Device according to one of Claims 7 to 9,
**characterized in that**
- the needles (20, 21, 22) or the rams (11) in each case constitute one electrical connection for the plasma source (15).

11. Device according to one of Claims 5 to 10,
**characterized in that**
- the at least one plasma source is arranged inside the chamber.

12. Device according to one of Claims 5 to 11,
**characterized in that**
- the transport device is connected to an electrical connection of the plasma source (15) or to a device for generating an electrical bias.

## Revendications

1. Procédé au moins pour stériliser des éléments d'obturation (12) de récipients,
selon lequel :
- dans au moins une étape du procédé, dans une chambre basse pression ou une chambre à vide (4) on applique un traitement par le plasma en excitant une oscillation électromagnétique et selon lequel
- le plasma (17, 47) est excité à proximité de l'élément d'obturation (12) ou d'un groupe d'éléments d'obturation (12) et d'un dispositif de transport (10 ; 20 ; 30 ; 40 ; 41 ; 43) pour les éléments d'obturation (12),
- on traite les zones du ou des éléments d'obturation (12) à stériliser, à dépyrogénéiser et/ou des zones à amender entre le sas d'entrée et le sas de sortie dans la chambre (4) par un mouvement dans et par le plasma,
- les éléments d'obturation (12) étant guidés séparément et stérilisés sur tous leurs côtés sur leur trajet à travers la chambre (4).

2. Procédé pour stériliser selon la revendication 1,
selon lequel :
- dans au moins une étape du procédé, dans une chambre basse pression ou une chambre à vide (4) on applique un traitement par le plasma commun à tous les récipients et à tous les éléments d'obturation (12) par excitation d'une oscillation électromagnétique,
- on excite le plasma (17, 47) à proximité de l'élément d'obturation (12) ou d'un groupe d'éléments d'obturation (12), d'un dispositif de transport (10, 20, 30, 40, 41, 43) et des récipients, et
- on traite par mouvement dans ou par le plasma les zones à stériliser, à dépyrogénéiser et/ou à amender du ou des éléments d'obturation (12) et des récipients entre le sas d'entrée et le sas de sortie dans la chambre (4).

3. Procédé pour stériliser selon la revendication 1,
selon lequel :
- dans au moins une étape du procédé, à chaque fois dans une chambre basse pression ou une chambre à vide (4) on effectue un traitement par le plasma des récipients et à chaque fois dans une autre chambre basse pression ou une chambre à vide (4) on traite les éléments d'obturation (12) par excitation d'une oscillation électromagnétique,
- on excite le plasma (17, 47) chaque fois à proximité de chaque élément d'obturation (12) ou groupe d'éléments d'obturation (12) et d'un dispositif de transport (10, 20, 30, 40, 41, 43) et chaque fois à proximité des récipients et d'un dispositif de transport, et
- on traite les zones à stériliser et/ou à dépyrogénéiser du ou des éléments d'obturation (12) et des récipients entre le sas d'entrée et le sas de sortie dans les chambres (4, 5) par un mouvement dans et par le plasma.

4. Procédé pour stériliser selon les revendications 1, 2 ou 3,
**caractérisé en ce qu'**
- avant d'introduire à travers un sas, les récipients et les éléments d'obturation (12) dans les chambres (4), on effectue un pré-nettoyage et/ou,
- après la sortie à travers un sas, des récipients et des éléments d'obturation (12) de la ou des chambres (4), on remplit (6) les récipients et on les ferme (7) à l'aide d'un élément d'obturation (12).

5. Dispositif pour stériliser des éléments d'obturation (12) de récipients selon un procédé de l'une des revendications précédentes, avec
- une source de plasma (15) pour générer un plasma nécessaire au traitement par du plasma,
- une chambre basse pression ou une chambre à vide (4) dans laquelle on effectue un traitement par le plasma en excitant une oscillation électromagnétique,
le plasma (17, 47) étant excité à proximité de l'élément d'obturation (12) ou d'un groupe d'éléments d'obturation (12) et d'un dispositif de transport (10),
selon lequel un guidage séparé des éléments d'obturation (12) étant prévu pour que les éléments d'obturation (12) soient stérilisés sur toutes leurs faces au cours de leur trajet à travers la chambre (4) et
selon lequel
dans la chambre, il y a le dispositif de transport (10) qui produit un mouvement du ou des éléments d'obturation (12) pour le transport entre le sas d'entrée (44) et le sas de sortie (46) dans la chambre et ainsi à travers le plasma,
selon lequel un nettoyage préalable séparé (2) est prévu pour les éléments d'obturation (12).

6. Dispositif pour stériliser selon la revendication 5,
**caractérisé en ce que**
- le dispositif de transport (10) comporte un piston (11) qui reçoit un élément d'obturation (12) respectif, le piston (11) constituant chaque fois un branchement électrique pour la source de plasma (15),
- un anneau (14) sur lequel s'appuie une partie en saillie périphérique de l'élément d'obturation (12) constitue l'autre branchement électrique de la source de plasma (15), et
- le piston (11) est mobile verticalement de façon que chaque fois en alternance, l'élément d'obturation (12) s'appuie soit sur le piston (11) soit sur l'anneau (14).

7. Dispositif pour stériliser selon la revendication 5,
**caractérisé en ce que**
- le dispositif de transport comporte un palier à aiguilles ou à coussinets (20) pour chaque élément d'obturation (12), et
- les aiguilles (21, 22) sur lesquelles s'appuie l'élément d'obturation (12) sont mobiles chaque fois par groupe, principalement dans la direction verticale et, chaque fois en alternance, l'élément d'obturation (12) s'appuie soit sur l'un des groupes (21) soit sur l'autre des groupes (22) d'aiguilles.

8. Dispositif pour stériliser selon la revendication 7,
**caractérisé en ce que**
les aiguilles sur lesquelles s'appuie l'élément d'obturation sont inclinées dans les groupes (21, 22) alternativement dans des directions opposées et elles sont déplacées alternativement de façon linéaire et/ou pour l'angle d'inclinaison de façon pour effectuer également une inclinaison et/ ou un mouvement de translation sur l'élément d'obturation (12)

9. Dispositif pour stériliser selon l'une des revendications 6 à 8,
**caractérisé en ce que**
pour fixer plusieurs éléments d'obturation (12), l'anneau (14) fait partie d'une tôle perforée, le piston (11) fait partie d'un coussin du piston ou les aiguilles (20, 21, 22) font partie d'un coussin d'aiguilles étendu.

10. Dispositif pour stériliser selon l'une des revendications 7 à 9,
**caractérisé en ce que**
les aiguilles (20, 21, 22) ou le piston (11) comportent respectivement un branchement électrique pour la source de plasma (15).

11. Dispositif pour stériliser selon l'une des revendications 5 à 10,
**caractérisé en ce qu'**
au moins une source de plasma est prévue à l'intérieur de la chambre.

12. Dispositif pour stériliser selon l'une des revendications 5 à 11,
**caractérisé en ce que**
le dispositif de transport est relié à un branchement électrique de la source de plasma (12) ou à une installation pour générer une tension électrique préalable.
